# EUROPEAN PATENT SPECIFICATION

(11) **EP 3 174 367 B1**
(45) Date of publication and mention of the grant of the patent: **01.06.2022**
(21) Application number: 17159273.6
(22) Date of filing: 03.03.2017
(51) Int. Cl.: H05B 3/26, A24F 47/00

(54) **HEATING ASSEMBLY AND CIGARETTE HEATING DEVICE HAVING SAME**
HEIZANORDNUNG UND ZIGARETTENERWÄRMUNGSRICHTUNG DAMIT
ENSEMBLE DE CHAUFFAGE ET DISPOSITIF DE CHAUFFAGE DE CIGARETTES EN ÉTANT DOTÉ

(30) Priority: 14.03.2016 CN 201620193100 U
(43) Date of publication of application: 31.05.2017
(73) Proprietor: Shenzhen First Union Technology Co., Ltd., Shenzhen, Guangdong 518104 (CN)
(72) Inventor: LI, Yonghai, Shenzhen Guangdong 518104 (CN); XU, Zhongli, Shenzhen Guangdong 518104 (CN); CHEN, Hongbin, Shenzhen Guangdong 518104 (CN); ZHONG, Yunping, Shenzhen Guangdong 518104 (CN); HUANG, Yanzhou, Shenzhen Guangdong 518104 (CN)
(74) Representative: Proi World Intellectual Property GmbH

(56) References cited:
- EP-A1- 2 394 520
- WO-A1-2012/117578
- WO-A1-2014/020953
- WO-A1-2015/150699
- CN-A- 104 585 884
- CN-U- 204 362 975
- US-A1- 2012 234 821
- US-A1- 2013 333 709
- US-A1- 2014 060 555

## Description

### TECHNICAL FIELD

The present invention relates to a heating assembly and a cigarette heating device using same.

### BACKGROUND ART

Atypical cigarette heating device includes a main body and a heating assembly. In the cigarette heating device, the heating assembly is integrally formed with the main body. The heating assembly is fragile since it always works in a high temperature environment. When the heating assembly is broken, it is inconvenient to replace the heating assembly with a new one, and thus the whole cigarette heating device should be discarded.

What are needed, therefore, are a heating assembly and a cigarette heating device using same, which can overcome the above shortcomings. Documents EP 2 394 520 A1, WO 2014/020953 A1, US 2012/234821 A1, US 2013/333709 A1, WO 2012/117578 A1, CN 104 585 884 A, WO 2015/150699 A1, US 2014/060555 A1 and CN 204 362 975 U disclose heating assemblies for cigarette heating devices according to the prior art.

### SUMMARY

The present disclosure relates to an exemplary heating assembly for a cigarette heating device. The cigarette heating device is configured for heating a cigarette. The heating assembly includes at least one heating head configured for heating the cigarette, a fixing holder configured for fixing the at least one heating head, and a plurality of electrodes arranged in the fixing holder. The fixing head is detachably connected with the cigarette heating device. The electrodes is electrically connected with the at least one heating head.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a perspective view of a heating assembly of a cigarette heating device according to an embodiment.
FIG. 2 is a side view of the cigarette heating device.
FIG. 3 is a cross-sectional view of the cigarette heating device.
FIG. 4 is an exploded view of the cigarette heating device together with a cigarette.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

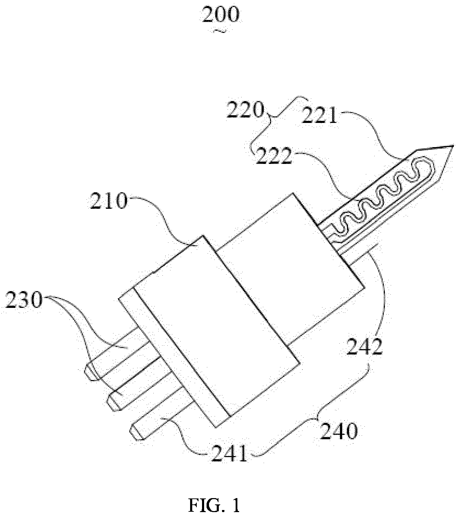

Referring to FIGS. 1-4, a heating assembly 200 is shown. The heating assembly 200 is detachably arranged in the heating device 10, and configured (i.e., structured and arranged) for heating a cigarette 400.

The heating assembly 200 includes a fixing holder 210, at least one heating head 220, and a plurality of electrodes 230. In other embodiments, the heating assembly 200 further includes a temperature sensing unit 240.

The fixing holder 210 is substantially cylindrical, and may be circular, or rectangular in cross-section. The fixing holder 210 is configured for fixing the heating head 220. The fixing holder 210 is detachably connected to the cigarette heating device 10. The fixing holder 210 is coupled with the main body 100 and the heating cup via interference fit or snap fit.

The heating head 220 is configured for heating a cigarette 400. In detail, the heating head 220 is arranged at an end of the fixing holder 210, and is inserted into the heating cup 300. Correspondingly, the heating cup 300 defines a through hole at a bottom part, which allows the heating part 220 to pass through.

Quite usefully, the heating head 220 includes a tip end at an end so that the heating end 220 can insert into a tobacco part 420 of the cigarette 400.

The heating head 220 includes a thermal conducting body 221 and a heating wire 222. The heating wire 222 may be, according to an example not being part of the claimed invention, formed inside of the thermal conducting body 221. According to the invention the heating wire 222 is printed on a surface of the thermal conducting body 221. Alternatively and in examples not being part of the claimed invention, the heating wire 222 may be wound around the thermal conducting body 221, or the thermal conducting body 221 may define a recess therein, and the heating wire 222 is arranged in the recess.

The heating assembly 200 may include one or more heating heads 220. Each heating head 220 may be plate shaped or cylindrical.

The electrodes 230 are arranged on the fixing holder 210, and are electrically connected with the heating head 220. The electrodes 230 may be formed at an end of the fixing holder 210 in a rod shape, as seen in FIG. 1. In other embodiments, the electrodes 230 may be formed on a side surface or an end surface of the fixing holder 210 in a form of a bump. Accordingly, the heating assembly 200 can be detachably and electrically connected with the main body 100 easily.

The electrodes 230 may include one or more groups. The groups of the electrodes 230 may be connected with the heating heads 220 in a one-to-one relationship. Alternatively, the heating heads 220 may be connected in series, and then connected to a group of electrodes.

The temperature sensing unit 240 includes an electrically conductive body 241 and a sensing head 242. The electrically conductive body 241 is arranged on a fixing holder 210 in a rod shape or a bump shape. The sensing head 242 is configured for sensing a temperature of the heating head 220. Alternatively, the sensing head 242 is in a form of a needle, and arranged at an end of the fixing holder 210. The sensing head 242 is configured for inserting the cigarette 400, and sensing a temperature of the cigarette 400.

The heating assembly 200 is pluggably arranged in the cigarette heating device 10, and thus can be detached or placed conveniently.

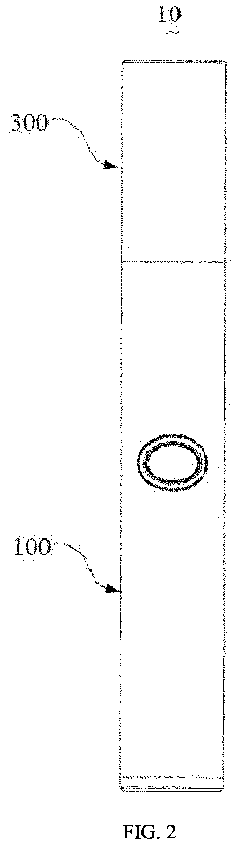

Referring to FIGS. 2-4, a cigarette heating device 10 is further provided. The cigarette heating device 10 is configured for heating a cigarette 400. The cigarette heating device 10 includes a main body 100, the heating assembly 200, and a heating cup 300.

The main body 100 includes a working circuit. The heating assembly 200 is detachably electrically connected with the working circuit of the main body. The heating cup 300 is detachably connected with the main body 100 or the heating assembly 200. The heating cup 300 is configured for receiving the cigarette 400.

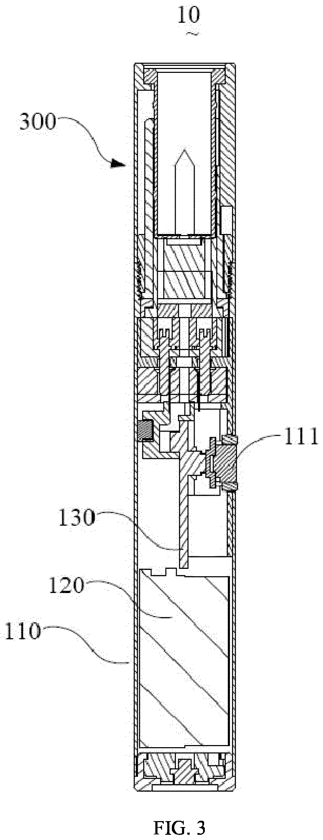

Referring to FIG. 3, the main body 100 includes a housing 110, and the working circuit. The working circuit includes a battery 120, a printed circuit board 130, and etc.

A control button 111 is provided on the housing 110, and the control button 111 may include a switch and a voltage adjusting button. The fixing holder 210 of the heating assembly 20 may be coupled with the housing 110 by interference fit or a snap-fit connection. The heating cup 300 is detachably connected with the housing 110 of the main body 100 or the fixing holder 210 of the heating assembly 200.

The battery 120 is arranged in the housing 110, and is configured for supplying the printed circuit board 130 and electronic components on the printed circuit board 130 power.

The printed circuit board 130 is arranged in the housing 110, and is electrically connected with the battery 120. The circuit board 130 may include a conductive inserting hole, a conductive pin, a conductive shrapnel, or a conductive card. The electrodes 230 and the electrically conductive body 241 are pluggably connected with the conductive inserting hole or the conductive pin. Alternatively, the electrodes 230 and the electrically conductive body 241 are in elastic contact with the conductive shrapnel or engaged with the conductive card by a snap-fit connection.

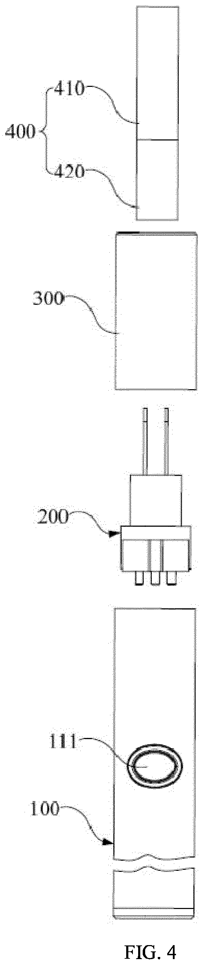

Referring to FIG. 4, the cigarette 400 includes a mouthpiece 410 and a tobacco part 420. Before use, the tobacco part 420 of the cigarette 400 is inserted into the heating cup 300, and is heated by the heating assembly 200. Correspondingly, the heating cup 300 defines a receiving chamber configured for receiving the cigarette 400. The heating cup 300 will not be described.

It is understood that the above-described embodiments are intended to illustrate rather than limit the disclosure.

## Claims

1. A heating assembly (200) for a cigarette heating device (10), the cigarette heating device being configured for heating a cigarette (400), the heating assembly comprising:
at least one heating head (220) configured for heating the cigarette;
a fixing holder (210) configured for fixing the at least one heating head, the fixing holder detachably connectable with the cigarette heating device; wherein the heating assembly further comprises a temperature sensing unit (240), the temperature sensing unit comprises an electrically conductive body (241) and a sensing head (242), the sensing head is arranged at an end of the fixing holder, and the sensing head is configured for sensing a temperature of the cigarette;
**characterised by**
a plurality of electrodes (230) arranged in the fixing holder, the electrodes being electrically connected with the at least one heating head,
wherein the at least one heating head comprises a thermal conducting body (221) and a heating wire (222), the heating wire is formed on a surface of the thermal conducting body by printing.

2. The heating assembly according to claim 1, wherein the at least one heating head comprises a tip end at a distal end.

3. The heating assembly according to claim 1, wherein the at least one heating head comprises a plurality of heating heads, and each heating head is plate-shaped or cylindrical.

4. The heating assembly according to claim 1, wherein the electrodes are arranged on the fixing holder in a rod shape, or formed on a surface of the fixing holder in a form of a bump.

5. The heating assembly according to claim 3, wherein the at least one heating head comprises a plurality of heating heads, the heating heads are connected in series, and then connected to the electrodes.

## Patentansprüche

1. Heizanordnung (200) für eine Zigarettenheizvorrichtung (10), wobei die Zigarettenheizvorrichtung zum Erhitzen einer Zigarette (400) konfiguriert ist, wobei die Heizanordnung umfasst:
mindestens einen Heizkopf (220), der zum Erhitzen der Zigarette konfiguriert ist;
einen Befestigungshalter (210), der zum Befestigen des mindestens einen Heizkopfes konfiguriert ist, wobei der Befestigungshalter lösbar mit der Zigarettenheizvorrichtung verbunden werden kann;
wobei die Heizanordnung ferner eine Temperaturerfassungseinheit (240) umfasst, wobei die Temperaturerfassungseinheit einen elektrisch leitenden Körper (241) und einen Erfassungskopf (242) umfasst, wobei der Erfassungskopf an einem Ende des Befestigungshalters angeordnet ist und der Erfassungskopf zum Erfassen einer Temperatur der Zigarette konfiguriert ist;
**gekennzeichnet durch**
eine Mehrzahl von Elektroden (230), die in der Befestigungshalterung angeordnet sind, wobei die Elektroden elektrisch mit dem mindestens einen Heizkopf verbunden sind, wobei der mindestens eine Heizkopf einen wärmeleitenden Körper (221) und einen Heizdraht (222) umfasst, wobei der Heizdraht auf einer Oberfläche des wärmeleitenden Körpers durch Drucken ausgebildet ist.

2. Heizanordnung nach Anspruch 1, wobei der mindestens eine Heizkopf an einem distalen Ende eine Spitze aufweist.

3. Heizanordnung nach Anspruch 1, wobei der mindestens eine Heizkopf eine Vielzahl von Heizköpfen umfasst und jeder Heizkopf plattenförmig oder zylindrisch ist.

4. Heizanordnung nach Anspruch 1, wobei die Elektroden auf dem Befestigungshalter stabförmig angeordnet oder auf einer Oberfläche des Befestigungshalters in Form eines Höckers ausgebildet sind.

5. Heizanordnung nach Anspruch 3, wobei der mindestens eine Heizkopf eine Vielzahl von Heizköpfen umfasst, die Heizköpfe in Reihe geschaltet und dann mit den Elektroden verbunden sind.

## Revendications

1. Ensemble de chauffage (200) pour un dispositif de chauffage de cigarettes (10), le dispositif de chauffage de cigarettes étant configuré pour chauffer une cigarette (400), l'ensemble de chauffage comprenant :
au moins une tête chauffante (220) configurée pour chauffer la cigarette ;
un support de fixation (210) configuré pour fixer la au moins une tête chauffante, le support de fixation pouvant être connecté de manière détachable au dispositif de chauffage de cigarette ;
dans lequel l'ensemble de chauffage comprend en outre une unité de détection de température (240), l'unité de détection de température comprend un corps électriquement conducteur (241) et une tête de détection (242), la tête de détection est disposée à une extrémité du support de fixation, et la tête de détection est configurée pour détecter une température de la cigarette ; **caractérisé par** une pluralité d'électrodes (230) disposées dans le support de fixation, les électrodes étant connectées électriquement à la au moins une tête chauffante, dans lequel la au moins une tête chauffante comprend un corps conducteur thermique (221) et un fil chauffant (222), le fil chauffant est formé sur une surface du corps conducteur thermique par impression.

2. Ensemble de chauffage selon la revendication 1, dans lequel la au moins une tête de chauffage comprend une extrémité de pointe à une extrémité distale.

3. Ensemble de chauffage selon la revendication 1, dans lequel la au moins une tête de chauffage comprend une pluralité de têtes de chauffage, et chaque tête de chauffage est en forme de plaque ou cylindrique.

4. Ensemble de chauffage selon la revendication 1, dans lequel les électrodes sont disposées sur le support de fixation en forme de tige, ou formées sur une surface du support de fixation en forme de bosse.

5. Ensemble de chauffage selon la revendication 3, dans lequel la au moins une tête chauffante comprend une pluralité de têtes chauffantes, les têtes chauffantes sont connectées en série, puis connectées aux électrodes.
